# EUROPEAN PATENT APPLICATION

(11) **EP 4 483 892 A1**
(43) Date of publication of application: **01.01.2025**
(21) Application number: 23182441.8
(22) Date of filing: 29.06.2023
(51) Int. Cl.: A61K 38/22, A61K 38/57, A61K 38/04, C07K 14/44

(54) **COMPOSITION COMPRISING LEKTI POLYPEPTIDES FOR THE TREATMENT OF DISORDERS**

(71) Applicant: Pharis Biotec GmbH, 30625 Hannover (DE)
(72) Inventor: Forssmann, Wolf-Georg, Hannover (DE)
(74) Representative: dompatent von Kreisler Selting Werner - Partnerschaft von Patent- und Rechtsanwälten mbB

(57) **Abstract**

Pharmaceutical composition comprising
a) an amino acid sequence from at least 70% sequence identity to the amino acid sequence of a polypeptide selected from at least one of the 15 domains of the LEKTI polypeptide;
and at least one selected from
b) an amino acid sequence from at least 75% sequence identity to the amino acid sequence of a polypeptide with antagonistic activities against natural CXCR4;
and
c) an amino acid sequence from at least 70% sequence identity to the amino acid sequence of a natriuretic peptide (ANP).

## Description

### Field of the invention

The present invention pertains to a pharmaceutical composition for the treatment of disorders.

### Background of the invention

An optimized derivative of an endogenous CXCR4 antagonist preventing atopic dermatitis and airway inflammation has been disclosed by Harms, M. et al. reports in Acta Pharmaceutica Sinica B 2021:11(9):2694-2708. The peptide molecule has been named EPI-X4. The polypeptide has also been disclosed in WO 2009/004054.

WO 02/066513 A2 and WO 03/070953 A1 reveal human circulating fragments of the serine proteinase inhibitor LEKTI and their use for the treatment of acute or chronic skin diseases, cervical inflammations, inflammations of the Bartholinian glands and other vaginal areas, tonsillitis, pharyngitis and laryngitis, acute or chronic inflammatory processes associated with excessive mucus formation and resulting acute emergency situations, postoperative bleeding due to hyperfibrinolysis, for the prophylaxis of pulmonary emphysema formation in α1-proteinase inhibitor deficiency, and for the therapy of asthma, AIDS, pneumonia, tumour disease and leukaemia. LEKTI is the acronym for Lympho-epithelial Kazal-type-related inhibitor (LEKTI) and also known as serine protease inhibitor Kazal-type 5 (SPINK5) and is encoded in humans by the gene SPINK5. WO 2021/198176 discloses the use of LEKTI for the treatment of COVID-19.

Atrial natriuretic peptide (ANP) belongs to the family of natriuretic peptides which comprise a family of three structurally related molecules. The other members are brain natriuretic peptide (BNP) and C-type natriuretic peptide (CNP), encoded by a gene symbolized NPPC. These peptides possess potent natriuretic, diuretic, and vasodilating activities and are implicated in body fluid homeostasis and blood pressure control.

### Summary of the Invention

It has been found that a pharmaceutical composition is useful in the treatment of neurological diseases, in particular stroke, Parkinson's disease, Alzheimer's disease, or multiple sclerosis; in the treatment of immunologic disorders, in particular in the treatment of the WHIm-syndrom, lupus erythematosus or rheumatoid arthritis; in the treatment of cancers, in particular sarcomas, lymphoma, leukaemia, or cancers showing the CRCX receptor such as cancer of the liver, pancreas, prostate, or breast cancer; in the treatment of dermatological diseases or inflammatory skin syndromes, in particular atopic dermatitis; in the treatment of lack of mobilization, proliferation and migration of stem cells, T-cell activation as well as support of immunoblasts such as CTL/PD-1; in the treatment of wounds caused by burning; in the treatment of antifibrosis; in the treatment or prevention of scars; in pain management; in the treatment of cardiologic disorders, in particular heart insufficiency; in the treatment of metabolic disorders, in particular diabetes; in the treatment of viral diseases, in particular COVID-19, infections with SARS-CoV-2, HIV-I, HIV-2, *Cytomegalo virus, Herpes simplex virus (type 1 and 2), Varicella zoster virus, Hepatitis A and Hepatitis B virus, Influenza virus, Polio virus, Rhino virus, Rubella virus, Measles virus, Rabies virus, Rous sarcoma virus,* or *Epstein-Barr Virus;* in the treatment of infections caused by bacteria and fungi, in particular *Pseudomonas, Candida,* or S. *aureus;* in the treatment of infectious processes or abnormal infectious processes; in the treatment of cervical inflammations, inflammations of the Bartholinian glands and other vaginal areas, tonsillitis, pharyngitis and laryngitis, acute or chronic inflammatory processes associated with excessive mucus formation and resulting acute emergency situations, or postoperative bleeding due to hyperfibrinolysis; in the prophylaxis of pulmonary emphysema formation in α1-proteinase inhibitor deficiency; in the treatment of asthma or pneumonia; in the treatment of growth disorders; in the treatment of neuronal diseases, disorders of the blood clotting cascade and hematopoiesis, vascular diseases, diseases of the immune system, organ infarcts, peripheral circulatory disorders, renal insufficiency, muscular degeneration, degenerative-genetic or acquired diseases, bone diseases, or in improving wound and bone healing, wherein the pharmaceutical composition is comprising a serine proteinase inhibitor LEKTI, and a peptide with antagonistic activities against natural CXCR4 or a atrial natriuretic peptide (ANP); or wherein the pharmaceutical composition is comprising a serine proteinase inhibitor LEKTI, a peptide with antagonistic activities against natural CXCR4, and a atrial natriuretic peptide (ANP).

The present invention therefore pertains to a pharmaceutical composition comprising
a) an amino acid sequence from at least 70% sequence identity to the amino acid sequence of a polypeptide selected from at least one of the 15 domains of the LEKTI polypeptide;
   and at least one selected from
b) an amino acid sequence from at least 75% sequence identity to the amino acid sequence of a polypeptide with antagonistic activities against natural CXCR4;
   and
c) an amino acid sequence from at least 70% sequence identity to the amino acid sequence of a natriuretic peptide (ANP).

In one embodiment of the invention, the polypeptide LEKTI, the polypeptide with antagonistic activity against natural CXCR4, or the polypeptide ANP independently of each other, may have a modification at their respective N-terminal nitrogen atoms which modification forms together with the amino group of the N-terminal amino acid of the peptide a moiety having the structure -NR2R3 wherein R2 and/or R3 are independently from each other H or a substituted or unsubstituted acyl alkyl, aryl, aralkyl, cyclo alkyl and heterocyclo alkyl group.

In another embodiment of the invention, the polypeptide LEKTI, the polypeptide with antagonistic activity against natural CXCR4, or the polypeptide ANP independently of each other, may have a modification at their respective C-terminal carboxyl group which modification forms together with the forms together with the carboxyl group of the C-terminal amino acid of the peptide a moiety having the structure -C(O)-O-R1 or -C(O)-NR2R3 wherein R1 is a substituted or unsubstituted alkyl, aryl, aralkyl, cyclo alkyl and heterocyclo alkyl group.

In another embodiment of the invention, the sequence identity to the LEKTI polypeptide amino acid sequence can be at least 80%, 90% or 95%,
and/or the sequence identity to the polypeptide sequence of the polypeptide with antagonistic activity against natural CXCR4 can be at least 80% or 90%,
and/or the sequence identity to the natriuretic polypeptide amino sequence can be at least 80%, 90% or 95%.

In yet another embodiment of the invention, the composition of the invention may comprise a mutant or derivative of the LEKTI polypeptide, a mutant or derivative of the peptide with antagonistic activity against natural CXCR4 polypeptide, or a mutant or derivative of the natriuretic peptide.

Also, retro-inverso peptides of the peptides of the invention are within the scope of the present invention, as well as other derivatives stabilizing the peptide bond against peptidases.

The term "derivative" means all length fragments including truncations at the N and C terminus, the peptide of the invention containing amino acid residue substitutions including D-amino acid residues and modified amino acid residues as well as peptides containing disulfide bonds and extension at the N and C terminus.

In a further embodiment, the pharmaceutical composition of the invention may have a biological activity of inhibiting tumour growth by at least 30 vol.-%, preferably by at least 40 vol.-%, more preferably by at least 50 vol.-%, most preferably by at least 90 vol.-%, determined in a patient-derived xenograft lymphoma model, respectively.

In still another embodiment, in the composition of the invention the polypeptide LEKTI can be selected from the group of polypeptides consisting of Seq ID No. 1 - 36.

In another embodiment, in the composition of the invention the polypeptide with antagonistic activity against natural CXCR4 polypeptide can be a peptide having the general amino acid sequence written in the single letter code
X¹ X² R X⁴ X⁵ X⁶ K X⁸ P X¹⁰ X¹¹ S,
wherein
X¹ = L or I,
X² = V, M, or L,
X⁴ = Y or W,
X⁵ = T or S,
X⁶ = K, C, R, or Q,
X⁸ = V, M, L, F, or A,
X¹⁰ = Q or C,
X¹¹ = V, M, or F.

In still another embodiment, in the composition of the invention the polypeptide with antagonistic activity against natural CXCR4 polypeptide may be selected from the group of EPI-X4 polypeptides consisting of Seq ID No. 37 - 52.

In yet another embodiment, in the composition of the invention the natriuretic peptide can be selected from the group of polypeptides consisting of Seq ID No. 53 - 55.

In another embodiment of the invention, the composition of the invention may comprise
a) an amino acid sequence of a polypeptide selected from the group of polypeptides consisting of Seq ID No 1 - 36;
   and at least one selected from
b) an amino acid sequence of a polypeptide selected from the group of EPI-X4 polypeptides consisting of Seq ID No 37 - 52;
   and
c) an amino acid sequence of a polypeptide selected from the group of polypeptides consisting of Seq ID No 53 - 55.

The composition of the invention can be formulated for intravascular, lymphatic, intracardial, parenteral, intravenous, intramuscular, intrathecal, pulmonary, epi-, intra- and sub-cutaneous, intranasal, ophthalmic, auricular, buccal, or intraperitoneal administration, or for local administration to an organ, or for administration into the cerebrospinal fluid space.

In one embodiment, the composition of the invention can comprise pharmaceutically acceptable carriers, stabilizers, or auxiliaries.

Subject matter of the invention is also the composition of the invention for use in the treatment of neurological diseases, in particular stroke, Parkinson's disease, Alzheimer's disease, or multiple sclerosis; in the treatment of immunologic disorders, in particular in the treatment of the WHIm-syndrom, lupus erythematosus or rheumatoid arthritis; in the treatment of cancers, in particular sarcomas, lymphoma, leukaemia, or cancers showing the CRCX receptor such as cancer of the liver, pancreas, prostate, or breast cancer; in the treatment of dermatological diseases or inflammatory skin syndromes, in particular atopic dermatitis; in the treatment of lack of mobilization, proliferation and migration of stem cells, T-cell activation as well as support of immunoblasts such as CTL/PD-1; in the treatment of wounds caused by burning; in the treatment of antifibrosis; in the treatment or prevention of scars; in pain management; in the treatment of cardiologic disorders, in particular heart insufficiency; in the treatment of metabolic disorders, in particular diabetes; in the treatment of viral diseases, in particular COVID-19, infections with SARS-CoV-2, HIV-I, HIV-2, *Cytomegalo virus, Herpes simplex virus (type 1 and 2), Varicella zoster virus, Hepatitis A and Hepatitis B virus, Influenza virus, Polio virus, Rhino virus, Rubella virus, Measles virus, Rabies virus, Rous sarcoma virus,* or *Epstein-Barr Virus;* in the treatment of infections caused by bacteria and fungi, in particular *Pseudomonas, Candida,* or *S*. *aureus;* in the treatment of infectious processes or abnormal infectious processes; in the treatment of cervical inflammations, inflammations of the Bartholinian glands and other vaginal areas, tonsillitis, pharyngitis and laryngitis, acute or chronic inflammatory processes associated with excessive mucus formation and resulting acute emergency situations, or postoperative bleeding due to hyperfibrinolysis; in the prophylaxis of pulmonary emphysema formation in α1-proteinase inhibitor deficiency; in the treatment of asthma or pneumonia; in the treatment of growth disorders; in the treatment of neuronal diseases, disorders of the blood clotting cascade and hematopoiesis, vascular diseases, diseases of the immune system, organ infarcts, peripheral circulatory disorders, renal insufficiency, muscular degeneration, degenerative-genetic or acquired diseases, bone diseases, or in improving wound and bone healing.

### Detailed description of the invention

The present invention concerns a pharmaceutical composition comprising
a) an amino acid sequence from at least 70% sequence identity to the amino acid sequence of a polypeptide selected from at least one of the 15 domains of the LEKTI polypeptide;
   and at least one selected from
b) an amino acid sequence from at least 75% sequence identity to the amino acid sequence of a polypeptide with antagonistic activities against natural CXCR4;
   and
c) an amino acid sequence from at least 70% sequence identity to the amino acid sequence of a natriuretic peptide (ANP).

In one embodiment of the invention, the polypeptide LEKTI, the polypeptide with antagonistic activity against natural CXCR4, or the polypeptide ANP independently of each other, are modified at their respective N-terminal nitrogen atoms. The respective modification forms together with the amino group of the N-terminal amino acid of the peptide a moiety having the structure -NR²R³ wherein R² and/or R³ are independently from each other H or a substituted or unsubstituted acyl alkyl, aryl, aralkyl, cyclo alkyl and heterocyclo alkyl group.

In another embodiment of the invention, the polypeptide LEKTI, the polypeptide with antagonistic activity against natural CXCR4, or the polypeptide ANP independently of each other, are modified at their respective C-terminal carboxyl group. The respective modification forms together with the carboxyl group of the C-terminal amino acid of the peptide a moiety having the structure -C(O)-O-R¹ or -C(O)-NR²R³ wherein R¹ is a substituted or unsubstituted alkyl, aryl, aralkyl, cyclo alkyl and heterocyclo alkyl group.

In another embodiment of the invention, the sequence identity to the LEKTI polypeptide amino acid sequence is at least 80%, 90% or 95%,
and/or the sequence identity to the polypeptide sequence of the polypeptide with antagonistic activity against natural CXCR4 is at least 80% or 90%,
and/or the sequence identity to the natriuretic polypeptide amino sequence is at least 80%, 90% or 95%.

In yet another embodiment of the invention, the composition of the invention may comprise a mutant or derivative of the LEKTI polypeptide, a mutant or derivative of the peptide with antagonistic activity against natural CXCR4 polypeptide, or a mutant or derivative of the natriuretic peptide.

Also, retro-inverso peptides of the peptides of the invention are within the scope of the present invention, as well as other derivatives stabilizing the peptide bond against peptidases.

The term "derivative" means all length fragments including truncations at the N and C terminus, the peptide of the invention containing amino acid residue substitutions including D-amino acid residues and modified amino acid residues as well as peptides containing disulfide bonds and extension at the N and C terminus.

In a further embodiment of the invention, the pharmaceutical composition of the invention has a biological activity of inhibiting tumour growth by at least 30 vol.- %, preferably by at least 40 vol.-%, more preferably by at least 50 vol.-%, most preferably by at least 90 vol.-%, determined in a patient-derived xenograft lymphoma model, respectively. The inhibition of the tumour growth is determined by determining the tumour volume after completion of the treatment relative to the tumour volume at the beginning of the treatment. E.g., if the tumour volume is halved during the treatment, which means that a stable disease is reached, the tumour growth is inhibited by 50 vol.-%.

The patient-derived xenograft (PDX) lymphoma model is the lymphoma model Ly13693. In this model, Ly13693 cells are derived form a patient with diffuse large B-cell lymphoma (Epstein-Barr virus (EBV)-associated; WHO classification: GCB (germinal centre B-cell-like). Solid tumours are first grown in donor mice and, thereafter, tumour tissues (about 1 cm³), free of necrotic or fibrotic tissue (macroscopic assessment) are retrieved from these mice. The tumours are cut into fragments (2-3 mm diameter - approximately 10 mm³) and placed in cooled RPMI 1640 culture medium until subcutaneous implantation within 30 minutes. The recipient mice are anesthetised and then a small incision is made in the skin of the left flank. The tumour fragments (one fragment/mouse) are implanted, and the incision is closed. When the animals attain a palpable tumour size of 100-200 mm, they are randomly divided into groups of 3 mice each and treated with the compositions which should be evaluated. Control groups may receive 0.9% NaCl (negative control) or rituximab, 10 mg/kg by i.v. injection (positive control), every second day. The compositions to be tested are administered e.g., over a period of 4 weeks. From the first day of treatment onwards, tumour diameters are measured twice weekly with a Vernier caliper. Tumour volumes are calculated according to V = (length × (width)²)/2. To calculate the relative tumour volume, the volumes of each measurement day are related to the day of the first treatment.

It is also possible to determine the biological activity of the pharmaceutical composition of the invention by determining the IC₅₀ value of the composition with respect to the CXCR-4 receptor activity. Methods of determining the IC₅₀ value of compositions with respect to a biological target, e.g., the CXCR-4 receptor, are known to the person skilled in the art.

In still another embodiment, in the composition of the invention the polypeptide LEKTI is selected from the group of polypeptides consisting of Seq ID No. 1 - 36.

In another embodiment, in the composition of the invention the polypeptide with antagonistic activity against natural CXCR4 polypeptide is a peptide having the general amino acid sequence written in the single letter code
X¹ X² R X⁴ X⁵ X⁶ K X⁸ P X¹⁰ X¹¹ S,
wherein
X¹ = L or I,
X² = V, M, or L,
X⁴ = Y or W,
X⁵ = T or S,
X⁶ = K, C, R, or Q,
X⁸ = V, M, L, F, or A,
X¹⁰ = Q or C,
X¹¹ = V, M, or F.

In another embodiment, in the composition of the invention the polypeptide with antagonistic activity against natural CXCR4 polypeptide is a peptide having the general amino acid sequence written in the single letter code
X¹ V RX⁴ X⁵ X⁶ K V P X¹⁰ V S,
wherein
X¹ = L or I,
X⁴ = Y or W,
X⁵ = T or S,
X⁶ = K or C,
X¹⁰ = Q or C.

In still another embodiment, in the composition of the invention the polypeptide with antagonistic activity against natural CXCR4 polypeptide is selected from the group of EPI-X4 polypeptides consisting of Seq ID No. 37 - 52.

In yet another embodiment, in the composition of the invention the natriuretic peptide is selected from the group of polypeptides consisting of Seq ID No. 53 - 55.

In another embodiment of the invention, the composition of the invention may comprise
a) an amino acid sequence of a polypeptide selected from the group of polypeptides consisting of Seq ID No 1 - 36;
   and at least one selected from
b) an amino acid sequence of a polypeptide selected from the group of EPI-X4 polypeptides consisting of Seq ID No 37 - 52;
   and
c) an amino acid sequence of a polypeptide selected from the group of polypeptides consisting of Seq ID No 53 - 55.

EPI-X4, LEKTI or ANP variants also include modified forms of the polypeptide as well as mutants or derivatives thereof. Modified EPI-X4, LEKTI or ANP are polypeptides in which one or more amino acids of the native sequence have been modified so that a non-naturally occurring amino acid residue is present in the polypeptide chain. Such modifications can in principle be performed during or after protein translation and include, for example, PEGylation, HESylation, phosphorylation, glycosylation, sulphonation, cross-linking, acylation, or proteolytic cleavage.

Amino acid substitutions can typically be made in a conservative manner. Conservative substitution refers to a mutation in which one codon is replaced by another. In this case, a different amino acid is encoded, which is, however, chemically related to the original amino acid, for example glycine to alanine or threonine to serine. A non-conservative substitution, on the other hand, occurs when the original codon is replaced by a codon encoding an amino acid with different chemical properties, for example glycine with lysine.

The terms "identical" or percent "identical" in the context of two or more polypeptides refer to two or more sequences or subsequences that are the same or have a certain percentage of amino acids that are identical when compared and "aligned" for maximum similarity using a sequence comparison algorithm. Optimised alignment for comparing sequences can be performed using, for example, the following algorithms: Local Homology Alignment of Smith &Waterman, Adv. Appl. Math. 2: 482 (1981), Homology Alignment Algorithm by Needleman & Wunsch, J. Mol.Biol. 48: 443 (1970), Pearson & Lipman, Proc. Nat'l. Acad.Sci. USA 85: 2444 (1988), (GAP, BESTFIT, FASTA, and TFASTA in Wisconsin Genetics Software Package, Genetics Computer Group, 575 Science Dr., Madison, WI), but also visual examination [compare, Current Protocols in Molecular Biology, (Ausubel, F. M. et al., eds.) John Wiley & Sons, Inc, New York (1987-1999, including Supplement 46 (April 1999)].

The expression "substantially identical" or similar expressions used in the context of two polypeptides refers to two or more sequences or subsequences that have at least 70%, in particular at least 80%, in particular at least 90%, in particular at least 95% sequence identity when compared and aligned for maximum similarity using a sequence comparison algorithm. For example, in case of the LEKTI polypeptide, substantial identity exists when there is a sequence region which is at least 40-60 amino acids in length, especially 60-80 amino acids, preferably more than 90-100 amino acid residues, and in particular is substantially identical over the entire length of the amino acid sequence of the native polypeptide. Fragments of a polypeptide can typically be obtained by cleaving the polypeptide chain of the corresponding domain of the EPI-X4, LEKTI or ANP polypeptide. Methods for fragmentation by cleavage of the polypeptide chain are known to the person skilled in the art.

For example, polypeptides can be cleaved by enzymes known as proteases. A distinction is made between proteinases (also called endopeptidases) depending on whether the proteases cleave polypeptides (proteins) within the amino acid chain or at the end. They cleave proteins within the amino acid chain. They mostly recognise specific sequence segments within a protein where they can attack and cleave there.

On the other hand, exoproteases (also called exopeptidases) cleave individual amino acids from the ends of the protein. In contrast to proteinases, they mainly break down smaller peptides and not proteins. A distinction is made between:
- Carboxypeptidases, which cleave amino acids from the carboxyl end (C-terminus) and
- Aminopeptidases that cleave amino acids from the amino end (N-terminus).

Specific proteases cleave the peptide bond between two specific amino acids; in some cases, they recognise more than one substrate. These proteases are used, for example, to investigate the affinity of proteins, to prepare proteins for sequencing and to isolate active domains of a protein. Some examples of such proteases are shown in Table 1 below:

**Table 1 - Specific proteases and their properties**

| Protease | Specificity of the cleavage |
|---|---|
| Pepsin | Phe⇓X, Leu⇓X and pairs of nonpolar amino acids |
| Trypsin | Arg⇓X, Lys⇓X |
| Chymotrypsin | Tyr⇓X, Phe⇓X, Trp⇓X |
| Thrombin | Arg⇓X |
| Thermolysin | X⇓Leu, X⇓Phe, other non-polar residues |
| Endoproteinase Lys-C | Lys ⇓X |
| Endoproteinase Glu-C | Glu⇓X (partly also Asp⇓X) |
| Endoproteinase Arg-C (clostripain) | Arg⇓X |

In contrast to specific proteases, non-specific proteases cleave the peptide chain before or after a whole series of amino acids and thus generate much smaller cleavage pieces. Some examples of such non-specific proteases are shown in Table 2 below:

**Table 2 - Non-specific proteases and their properties**

| Protease | Specificity of the cleavage |
|---|---|
| Alkaline protease | Phe⇓X, Leu⇓X, Val⇓X, Ile⇓X, Trp⇓X, Tyr⇓X |
| Papaine | Arg⇓X, Lys⇓X, Phe⇓X |
| Proteinase K | Broad substrate range |

Certain chemicals can be used for the proteolytic analysis of peptides in addition to proteases. Cyanogen bromide (BrCN) is the most important reagent of this type and cleaves proteins on the C-terminal side of methionine residues. Peptidylhomoserine lactone is formed in the process. The resulting peptide fragments can then be separated in polyacrylamide gel and visualised by means of staining.

**Table 3 - Typical reagents for chemical proteolysis and their properties**

| Reagent | Specificity of the cleavage |
|---|---|
| Cyanogen bromide | Met⇓X |
| Partial acid hydrolysis | Asp⇓Pro |
| Hydroxylamine | Asn⇓Gly |

A special case of proteolysis is what is known as limited proteolysis. In contrast to total hydrolysis, protein digestion is not complete in this method, but takes place under precisely defined reaction conditions (proteolysis thus proceeds in a limited manner). In the globular regions of a native protein, the peptide bonds are much less exposed than the peptide bonds on the surface of the protein. This means that if a protease is applied for a short time, or if the protease concentration is very low, the individual protein domains may be separated first before the protease reaches cleavage sites further inside. This means that limited proteolysis is carried out with protease dilution and/or suboptimal reaction conditions and is stopped after a short time. In this method, the protein is cleaved proteolytically in its native form (and not after denaturation); this sometimes has consequences for the choice of protease. Metalloprotease cannot be used for digestion if the protein requires e.g. EDTA for optimum stability.

Further information on protein analysis and procedures for proteolytic cleavage of polypeptides can be found in the textbook "Arbeitsmethoden der Biochemie" ('Working methods in biochemistry') by Alfred Pingoud, Claus Urbanke, Walter de Gruyter, for example chapter 5.1.6.

In a further embodiment of the invention, the polypeptide selected from at least one of the 15 domains of the LEKTI polypeptide has at least 80%, in particular at least 90%, in particular at least 95% sequence identity with one of the domains 1 to 14 of the LEKTI polypeptide, especially from the group consisting of SEQ ID Nos 1 to 36. Preferably, said polypeptide has at least 80%, in particular at least 90%, in particular at least 95% sequence identity with one of the domains 2 to 14 of the LEKTI, polypeptide, especially from the group consisting of SEQ ID Nos 2 to 36. Particularly, said polypeptide has at least 80%, in particular at least 90%, in particular at least 95% sequence identity with one of the domains 3 to 14 of the LEKTI polypeptide, especially from the group consisting of SEQ ID Nos 3 to 36.

In another embodiment of the invention, the pharmaceutical composition is formulated for intravascular, lymphatic, intracardial, parenteral, intravenous, intramuscular, intrathecal, pulmonary, epi-, intra- and sub-cutaneous, intranasal, ophthalmic, auricular, buccal, or intraperitoneal administration, or for local administration to an organ, or for administration into the cerebrospinal fluid space. These routes of administration of pharmaceutical compounds and compositions and their execution are known to the person skilled in the art. The intravascular administration may be an administration into local vessels or general vascularisation. Preferably, the pharmaceutical composition is formulated for pulmonary, intranasal, ophthalmic, or auricular administration or for administration into the cerebrospinal fluid space.

In another preferred embodiment, the pharmaceutical composition is formulated for parenteral administration.

In still another embodiment of the invention, the pharmaceutical composition of the invention comprises pharmaceutically acceptable carriers, such as a pharmaceutically acceptable gas, a sodium chloride solution, or a citrate/bicarbonate buffer, stabilizers, such as sugars selected from the group consisting of mannitol, sucrose, and lactose, preferably mannitol, and/or auxiliaries. Suitable pharmaceutically acceptable carriers, stabilizers, and auxiliaries according to Pharmacopoeia Europaea (Ph. Eur.) are known to the person skilled in the art.

In still another embodiment, the polypeptides are present in the pharmaceutical composition of the invention in substantially aqueous solution, in particular in aqueous solution with pharmaceutical excipients.

The excipients are typically added individually or in combination not only to the polypeptide but also to a final formulation. The excipients may be added at various points in the galenical preparation of the medicinal product.

It may be advisable to stabilise the polypeptides in the composition of the invention against aggregation or oligomerisation. If necessary, a bacteriostatic agent such as benzyl alcohol, a surfactant such as Tween 20, a stabilizer such as mannitol, one or more stabilising amino acids such as lysine or arginine, and an antioxidant may also be added to the composition of the invention.

The pharmaceutical composition of the present invention may be used in the treatment of neurological diseases, in particular stroke, Parkinson's disease, Alzheimer's disease, or multiple sclerosis; in the treatment of immunologic disorders, in particular in the treatment of the WHIm-syndrom, lupus erythematosus or rheumatoid arthritis; in the treatment of cancers, in particular sarcomas, lymphoma, leukaemia, or cancers showing the CRCX receptor such as cancer of the liver, pancreas, prostate, or breast cancer; in the treatment of dermatological diseases or inflammatory skin syndromes, in particular atopic dermatitis; in the treatment of lack of mobilization, proliferation and migration of stem cells, T-cell activation as well as support of immunoblasts such as CTL/PD-1; in the treatment of wounds caused by burning; in the treatment of antifibrosis; in the treatment or prevention of scars; in pain management; in the treatment of cardiologic disorders, in particular heart insufficiency; in the treatment of metabolic disorders, in particular diabetes; in the treatment of viral diseases, in particular COVID-19, infections with SARS-CoV-2, HIV-I, HIV-2, *Cytomegalo virus, Herpes simplex virus (type 1 and 2), Varicella zoster virus, Hepatitis A and Hepatitis B virus, Influenza virus, Polio virus, Rhino virus, Rubella virus, Measles virus, Rabies virus, Rous sarcoma virus,* or *Epstein-Barr Virus;* in the treatment of infections caused by bacteria and fungi, in particular *Pseudomonas, Candida,* or *S*. *aureus;* in the treatment of infectious processes or abnormal infectious processes; in the treatment of cervical inflammations, inflammations of the Bartholinian glands and other vaginal areas, tonsillitis, pharyngitis and laryngitis, acute or chronic inflammatory processes associated with excessive mucus formation and resulting acute emergency situations, or postoperative bleeding due to hyperfibrinolysis; in the prophylaxis of pulmonary emphysema formation in a1-proteinase inhibitor deficiency; in the treatment of asthma or pneumonia; in the treatment of growth disorders; in the treatment of neuronal diseases, disorders of the blood clotting cascade and hematopoiesis, vascular diseases, diseases of the immune system, organ infarcts, peripheral circulatory disorders, renal insufficiency, muscular degeneration, degenerative-genetic or acquired diseases, bone diseases, or in improving wound and bone healing.

SEQUENCE LISTING

Polypeptide LEKTI

SEQ ID NO 1:
Lys Asn Glu Asp Gln Glu Met Cys His Glu Phe Gln Ala Phe Met Lys
1               5                   10                  15
Asn Gly Lys Leu Phe Cys Pro Gln Asp Lys Lys Phe Phe Gln Ser Leu
            20                  25                  30
Asp Gly Ile Met Phe Ile Asn Lys Cys Ala Thr Cys Lys Met Ile Leu
        35                  40                  45
Glu Lys Glu Ala Lys Ser Gln Lys Arg
    50                  55

SEQ ID NO 2:
Ala Arg His Leu Ala Arg Ala Pro Lys Ala Thr Ala Pro Thr Glu Leu
1               5                   10                  15
Asn Cys Asp Asp Phe Lys Lys Gly Glu Arg Asp Gly Asp Phe Ile Cys
            20                  25                  30
Pro Asp Tyr Tyr Glu Ala Val Cys Gly Thr Asp Gly Lys Thr Tyr Asp
        35                  40                  45
Asn Arg Cys Ala Leu Cys Ala Glu Asn Ala Lys Thr Gly Ser Gln Ile
    50                  55                  60
Gly Val Lys Ser Glu Gly Glu Cys Lys Ser Ser Asn
65                  70                  75

SEQ ID NO 3:
Pro Glu Gln Asp Val Cys Ser Ala Phe Arg Pro Phe Val Arg Asp Gly
1               5                   10                  15
Arg Leu Gly Cys Thr Arg Glu Asn Asp Pro Val Leu Gly Pro Asp Gly
            20                  25                  30
Lys Thr His Gly Asn Lys Cys Ala Met Cys Ala Glu Leu Phe Leu Lys
        35                  40                  45
Glu Ala Glu Asn Ala Lys Arg Glu Gly Glu Thr Arg Ile Arg Arg Asn
    50                  55                  60

SEQ ID NO 4:
Pro Glu Gln Asp Val Cys Ser Ala Phe Arg Pro Phe Val Arg Asp Gly
1               5                   10                  15
Arg Leu Gly Cys Thr Arg Glu Asn Asp Pro Val Cys Gly Pro Asp Gly
            20                  25                  30
Lys Thr His Gly Asn Lys Cys Ala Met Cys Ala Glu Leu Phe Leu Lys
        35                  40                  45
Glu Ala Glu Asn Ala Lys Arg Glu Gly Glu Thr Cys Ile Arg Arg Asn
    50                  55                  60

SEQ ID NO 5:
Ala Glu Lys Asp Phe Cys Lys Glu Tyr Glu Lys Gln Val Arg Asn Gly
1               5                   10                  15
Arg Leu Phe Cys Thr Arg Glu Ser Asp Pro Val Arg Gly Pro Asp Gly
            20                  25                  30
Arg Met His Gly Asn Lys Cys Ala Leu Cys Ala Glu Ile Phe Lys Gln
        35                  40                  45
Arg Phe Ser Glu Glu Asn Ser Lys Thr Asp Gln Asn Leu Gly Lys Ala
    50                  55                  60
Glu Glu Lys Thr Lys Val Lys Arg
65                  70

SEQ ID NO 6:
Ala Glu Lys Asp Phe Cys Lys Glu Tyr Glu Lys Gln Val Arg Asn Gly
1               5                   10                  15
Arg Leu Phe Cys Thr Arg Glu Ser Asp Pro Val Cys Gly Pro Asp Gly
            20                  25                  30
Arg Met His Gly Asn Lys Cys Ala Leu Cys Ala Glu Ile Phe Lys Gln
        35                  40                  45
Arg Phe Ser Glu Glu Asn Ser Lys Thr Asp Gln Cys Leu Gly Lys Ala
    50                  55                  60
Glu Glu Lys Thr Lys Val Lys Arg
65                  70

SEQ ID NO 7:
Ala Glu Lys Asp Phe Cys Lys Glu Tyr Glu Lys Gln Val Arg Asn Gly
1               5                   10                  15
Arg Leu Phe Cys Thr Arg Glu Ser Asp Pro Val Arg Gly Pro Asp Gly
            20                  25                  30
Arg Met His Gly Asn Lys Cys Ala Leu Cys Ala Glu Ile Phe Lys Arg
        35                  40                  45
Arg Phe Ser Glu Glu Asn Ser Lys Thr Asp Gln Asn Leu Gly Lys Ala
    50                  55                  60
Glu Glu Lys Thr Lys Val Lys Arg
65                  70

SEQ ID NO 8:
Ala Glu Lys Asp Phe Cys Lys Glu Tyr Glu Lys Gln Val Arg Asn Gly
1               5                   10                  15
Arg Leu Phe Cys Thr Arg Glu Ser Asp Pro Val Cys Gly Pro Asp Gly
            20                  25                  30
Arg Met His Gly Asn Lys Cys Ala Leu Cys Ala Glu Ile Phe Lys Arg
        35                  40                  45
Arg Phe Ser Glu Glu Asn Ser Lys Thr Asp Gln Cys Leu Gly Lys Ala
    50                  55                  60
Glu Glu Lys Thr Lys Val Lys Arg
65                  70

SEQ ID NO 9:
Glu Ile Val Lys Leu Cys Ser Gln Tyr Gln Asn Gln Ala Lys Asn Gly
1               5                   10                  15
Ile Leu Phe Cys Thr Arg Glu Asn Asp Pro Ile Arg Gly Pro Asp Gly
            20                  25                  30
Lys Met His Gly Asn Leu Cys Ser Met Cys Gln Ala Tyr Phe Gln Ala
        35                  40                  45
Glu Asn Glu Glu Lys Lys Lys Ala Glu Ala Arg Ala Arg Asn Lys Arg
    50                  55                  60

SEQ ID NO 10:
Glu Ile Val Lys Leu Cys Ser Gln Tyr Gln Asn Gln Ala Lys Asn Gly
1               5                   10                  15
Ile Leu Phe Cys Thr Arg Glu Asn Asp Pro Ile Cys Gly Pro Asp Gly
            20                  25                  30
Lys Met His Gly Asn Leu Cys Ser Met Cys Gln Ala Tyr Phe Gln Ala
        35                  40                  45
Glu Asn Glu Glu Lys Lys Lys Ala Glu Ala Arg Cys Arg Asn Lys Arg
    50                  55                  60

SEQ ID NO 11:
Glu Ser Gly Lys Ala Thr Ser Tyr Ala Glu Leu Cys Ser Glu Tyr Arg
1               5                   10                  15
Lys Leu Val Arg Asn Gly Lys Leu Ala Cys Thr Arg Glu Asn Asp Pro
            20                  25                  30
Ile Gln Gly Pro Asp Gly Lys Val His Gly Asn Thr Cys Ser Met Cys
        35                  40                  45
Glu Val Phe Phe Gln Ala Glu Glu Glu Glu Lys Lys Lys Lys Glu Gly
    50                  55                  60
Lys Ser Arg Asn Lys Arg
65                  70

SEQ ID NO 12:
Glu Ser Gly Lys Ala Thr Ser Tyr Ala Glu Leu Cys Ser Glu Tyr Arg
1               5                   10                  15
Lys Leu Val Arg Asn Gly Lys Leu Ala Cys Thr Arg Glu Asn Asp Pro
            20                  25                  30
Ile Cys Gly Pro Asp Gly Lys Val His Gly Asn Thr Cys Ser Met Cys
        35                  40                  45
Glu Val Phe Phe Gln Ala Glu Glu Glu Glu Lys Lys Lys Lys Glu Gly
    50                  55                  60
Lys Cys Arg Asn Lys Arg
65                  70

SEQ ID NO 13:
Glu Ser Gly Lys Ala Thr Ser Tyr Ala Glu Leu Cys Asn Glu Tyr Arg
1               5                   10                  15
Lys Leu Val Arg Asn Gly Lys Leu Ala Cys Thr Arg Glu Asn Asp Pro
            20                  25                  30
Ile Gln Gly Pro Asp Gly Lys Val His Gly Asn Thr Cys Ser Met Cys
        35                  40                  45
Glu Val Phe Phe Gln Ala Glu Glu Glu Glu Lys Lys Lys Lys Glu Gly
    50                  55                  60
Lys Ser Arg Asn Lys Arg
65                  70

SEQ ID NO 14:
Glu Ser Gly Lys Ala Thr Ser Tyr Ala Glu Leu Cys Asn Glu Tyr Arg
1               5                   10                  15
Lys Leu Val Arg Asn Gly Lys Leu Ala Cys Thr Arg Glu Asn Asp Pro
            20                  25                  30
Ile Cys Gly Pro Asp Gly Lys Val His Gly Asn Thr Cys Ser Met Cys
        35                  40                  45
Glu Val Phe Phe Gln Ala Glu Glu Glu Glu Lys Lys Lys Lys Glu Gly
    50                  55                  60
Lys Cys Arg Asn Lys Arg
65                  70

SEQ ID NO 15:
Glu Ser Gly Lys Ala Thr Ser Tyr Ala Glu Leu Cys Ser Glu Tyr Arg
1               5                   10                  15
Lys Leu Val Arg Asn Gly Lys Leu Ala Cys Thr Arg Glu Asn Asp Pro
            20                  25                  30
Ile Gln Gly Pro Asp Gly Lys Val His Gly Asn Thr Cys Ser Met Cys
        35                  40                  45
Glu Val Phe Phe Gln Ala Glu Glu Glu Glu Lys Lys Lys Lys Glu Gly
    50                  55                  60
Glu Ser Arg Asn Lys Arg
65                  70

SEQ ID NO 16:
Glu Ser Gly Lys Ala Thr Ser Tyr Ala Glu Leu Cys Ser Glu Tyr Arg
1               5                   10                  15
Lys Leu Val Arg Asn Gly Lys Leu Ala Cys Thr Arg Glu Asn Asp Pro
            20                  25                  30
Ile Cys Gly Pro Asp Gly Lys Val His Gly Asn Thr Cys Ser Met Cys
        35                  40                  45
Glu Val Phe Phe Gln Ala Glu Glu Glu Glu Lys Lys Lys Lys Glu Gly
    50                  55                  60
Glu Cys Arg Asn Lys Arg
65                  70

SEQ ID NO 17:
Glu Ser Gly Lys Ala Thr Ser Tyr Ala Glu Leu Cys Asn Glu Tyr Arg
1               5                   10                  15
Lys Leu Val Arg Asn Gly Lys Leu Ala Cys Thr Arg Glu Asn Asp Pro
            20                  25                  30
Ile Gln Gly Pro Asp Gly Lys Val His Gly Asn Thr Cys Ser Met Cys
        35                  40                  45
Glu Val Phe Phe Gln Ala Glu Glu Glu Glu Lys Lys Lys Lys Glu Gly
    50                  55                  60
Glu Ser Arg Asn Lys Arg
65                  70

SEQ ID NO 18:
Glu Ser Gly Lys Ala Thr Ser Tyr Ala Glu Leu Cys Asn Glu Tyr Arg
1               5                   10                  15
Lys Leu Val Arg Asn Gly Lys Leu Ala Cys Thr Arg Glu Asn Asp Pro
            20                  25                  30
Ile Cys Gly Pro Asp Gly Lys Val His Gly Asn Thr Cys Ser Met Cys
        35                  40                  45
Glu Val Phe Phe Gln Ala Glu Glu Glu Glu Lys Lys Lys Lys Glu Gly
    50                  55                  60
Glu Cys Arg Asn Lys Arg
65                  70

SEQ ID NO 19:
Gln Ser Lys Ser Thr Ala Ser Phe Glu Glu Leu Cys Ser Glu Tyr Arg
1               5                   10                  15
Lys Ser Arg Lys Asn Gly Arg Leu Phe Cys Thr Arg Glu Asn Asp Pro
            20                  25                  30
Ile Gln Gly Pro Asp Gly Lys Met His Gly Asn Thr Cys Ser Met Cys
        35                  40                  45
Glu Ala Phe Phe Gln Gln Glu Glu Arg Ala Arg Ala Lys Ala Lys Arg
    50                  55                  60

SEQ ID NO 20:
Glu Ala Ala Lys Glu Ile Cys Ser Glu Phe Arg Asp Gln Val Arg Asn
1               5                   10                  15
Gly Thr Leu Ile Cys Thr Arg Glu His Asn Pro Val Arg Gly Pro Asp
            20                  25                  30
Gly Lys Met His Gly Asn Lys Cys Ala Met Cys Ala Ser Val Phe Lys
        35                  40                  45
Leu Glu Glu Glu Glu Lys Lys Asn Asp Lys Glu Glu Lys Gly Lys Val
    50                  55                  60
Glu Ala Glu Lys Val Lys Arg
65                  70

SEQ ID NO 21:
Glu Ala Ala Lys Glu Ile Cys Ser Glu Phe Arg Asp Gln Val Arg Asn
1               5                   10                  15
Gly Thr Leu Ile Cys Thr Arg Glu His Asn Pro Val Cys Gly Pro Asp
            20                  25                  30
Gly Lys Met His Gly Asn Lys Cys Ala Met Cys Ala Ser Val Phe Lys
        35                  40                  45
Leu Glu Glu Glu Glu Lys Lys Asn Asp Lys Glu Glu Cys Gly Lys Val
    50                  55                  60
Glu Ala Glu Lys Val Lys Arg
65                  70

SEQ ID NO 22:
Glu Ala Val Gln Glu Leu Cys Ser Glu Tyr Arg His Tyr Val Arg Asn
1               5                   10                  15
Gly Arg Leu Pro Cys Thr Arg Glu Asn Asp Pro Ile Glu Gly Leu Asp
            20                  25                  30
Gly Lys Ile His Gly Asn Thr Cys Ser Met Cys Glu Ala Phe Phe Gln
        35                  40                  45
Gln Glu Ala Lys Glu Lys Glu Arg Ala Glu Pro Arg Ala Lys Val Lys
    50                  55                  60
Arg
65

SEQ ID NO 23:
Glu Ala Val Gln Glu Leu Cys Ser Glu Tyr Arg His Tyr Val Arg Asn
1               5                   10                  15
Gly Arg Leu Pro Cys Thr Arg Glu Asn Asp Pro Ile Cys Gly Leu Asp
            20                  25                  30
Gly Lys Ile His Gly Asn Thr Cys Ser Met Cys Glu Ala Phe Phe Gln
        35                  40                  45
Gln Glu Ala Lys Glu Lys Glu Arg Ala Glu Pro Arg Cys Lys Val Lys
    50                  55                  60
Arg
65

SEQ ID NO 24:
Glu Ala Glu Lys Glu Thr Cys Asp Glu Phe Arg Arg Leu Leu Gln Asn
1               5                   10                  15
Gly Lys Leu Phe Cys Thr Arg Glu Asn Asp Pro Val Arg Gly Pro Asp
            20                  25                  30
Gly Lys Thr His Gly Asn Lys Cys Ala Met Cys Lys Ala Val Phe Gln
        35                  40                  45
Lys Glu Asn Glu Glu Arg Lys Arg Lys Glu Glu Glu Asp Gln Arg Asn
    50                  55                  60

SEQ ID NO 25:
Glu Ala Glu Lys Glu Thr Cys Asp Glu Phe Arg Arg Leu Leu Gln Asn
1               5                   10                  15
Gly Lys Leu Phe Cys Thr Arg Glu Asn Asp Pro Val Cys Gly Pro Asp
            20                  25                  30
Gly Lys Thr His Gly Asn Lys Cys Ala Met Cys Lys Ala Val Phe Gln
        35                  40                  45
Lys Glu Asn Glu Glu Arg Lys Arg Lys Glu Glu Glu Cys Gln Arg Asn
    50                  55                  60

SEQ ID NO 26:
Ala Ala Gly His Gly Ser Ser Gly Gly Gly Gly Gly Asn Thr Gln Asp
1               5                   10                  15
Glu Cys Ala Glu Tyr Arg Glu Gln Met Lys Asn Gly Arg Leu Ser Cys
            20                  25                  30
Thr Arg Glu Ser Asp Pro Val Arg Asp Ala Asp Gly Lys Ser Tyr Asn
        35                  40                  45
Asn Gln Cys Thr Met Cys Lys Ala Lys Leu Glu Arg Glu Ala Glu Arg
    50                  55                  60
Lys Asn Glu Tyr Ser Arg Ser Arg Ser Asn
65                  70

SEQ ID NO 27:
Ala Ala Gly His Gly Ser Ser Gly Gly Gly Gly Gly Asn Thr Gln Asp
1               5                   10                  15
Glu Cys Ala Glu Tyr Arg Glu Gln Met Lys Asn Gly Arg Leu Ser Cys
            20                  25                  30
Thr Arg Glu Ser Asp Pro Val Cys Asp Ala Asp Gly Lys Ser Tyr Asn
        35                  40                  45
Asn Gln Cys Thr Met Cys Lys Ala Lys Leu Glu Arg Glu Ala Glu Arg
    50                  55                  60
Lys Asn Glu Tyr Ser Arg Ser Cys Ser Asn
65                  70

SEQ ID NO 28:
Ala Ala Gly His Gly Ser Ser Gly Gly Gly Gly Gly Asn Thr Gln Asp
1               5                   10                  15
Glu Cys Ala Glu Tyr Gln Glu Gln Met Lys Asn Gly Arg Leu Ser Cys
            20                  25                  30
Thr Arg Glu Ser Asp Pro Val Arg Asp Ala Asp Gly Lys Ser Tyr Asn
        35                  40                  45
Asn Gln Cys Thr Met Cys Lys Ala Lys Leu Glu Arg Glu Ala Glu Arg
    50                  55                  60
Lys Asn Glu Tyr Ser Arg Ser Arg Ser Asn
65                  70

SEQ ID NO 29:
Ala Ala Gly His Gly Ser Ser Gly Gly Gly Gly Gly Asn Thr Gln Asp
1               5                   10                  15
Glu Cys Ala Glu Tyr Gln Glu Gln Met Lys Asn Gly Arg Leu Ser Cys
            20                  25                  30
Thr Arg Glu Ser Asp Pro Val Cys Asp Ala Asp Gly Lys Ser Tyr Asn
        35                  40                  45
Asn Gln Cys Thr Met Cys Lys Ala Lys Leu Glu Arg Glu Ala Glu Arg
    50                  55                  60
Lys Asn Glu Tyr Ser Arg Ser Cys Ser Asn
65                  70

SEQ ID NO 30:
Gly Thr Gly Ser Glu Ser Gly Lys Asp Thr Cys Asp Glu Phe Arg Ser
1               5                   10                  15
Gln Met Lys Asn Gly Lys Leu Ile Cys Thr Arg Glu Ser Asp Pro Val
            20                  25                  30
Arg Gly Pro Asp Gly Lys Thr His Gly Asn Lys Cys Thr Met Cys Lys
        35                  40                  45
Glu Lys Leu Glu Arg Glu Ala Ala Glu Lys Lys Lys Lys Glu Asp Glu
    50                  55                  60
Asp Arg Ser Asn
65

SEQ ID NO 31:
Gly Thr Gly Ser Glu Ser Gly Lys Asp Thr Cys Asp Glu Phe Arg Ser
1               5                   10                  15
Gln Met Lys Asn Gly Lys Leu Ile Cys Thr Arg Glu Ser Asp Pro Val
            20                  25                  30
Cys Gly Pro Asp Gly Lys Thr His Gly Asn Lys Cys Thr Met Cys Lys
        35                  40                  45
Glu Lys Leu Glu Arg Glu Ala Ala Glu Lys Lys Lys Lys Glu Asp Glu
    50                  55                  60
Cys Arg Ser Asn
65

SEQ ID NO 32:
Thr Gly Glu Arg Ser Asn Thr Gly Glu Arg Ser Asn Asp Lys Glu Asp
1               5                   10                  15
Leu Cys Arg Glu Phe Arg Ser Met Gln Arg Asn Gly Lys Leu Ile Cys
            20                  25                  30
Thr Arg Glu Asn Asn Pro Val Arg Gly Pro Tyr Gly Lys Met His Ile
        35                  40                  45
Asn Lys Cys Ala Met Cys Gln Ser Ile Phe Asp Arg Glu Ala Asn Glu
    50                  55                  60
Arg Lys Lys Lys Asp Glu Glu Lys Ser Ser Ser Lys Pro Ser Asn
65                  70                  75

SEQ ID NO 33:
Thr Gly Glu Arg Ser Asn Thr Gly Glu Arg Ser Asn Asp Lys Glu Asp
1               5                   10                  15
Leu Cys Arg Glu Phe Arg Ser Met Gln Arg Asn Gly Lys Leu Ile Cys
            20                  25                  30
Thr Arg Glu Asn Asn Pro Val Cys Gly Pro Tyr Gly Lys Met His Ile
        35                  40                  45
Asn Lys Cys Ala Met Cys Gln Ser Ile Phe Asp Arg Glu Ala Asn Glu
    50                  55                  60
Arg Lys Lys Lys Asp Glu Glu Cys Ser Ser Ser Lys Pro Ser Asn
65                  70                  75

SEQ ID NO 34:
Asn Ala Lys Asp Glu Cys Ser Glu Phe Arg Asn Tyr Ile Arg Asn Asn
1               5                   10                  15
Glu Leu Ile Cys Pro Arg Glu Asn Asp Pro Val His Gly Ala Asp Gly
            20                  25                  30
Lys Phe Tyr Thr Asn Lys Cys Tyr Met Cys Arg Ala Val Phe Leu Thr
        35                  40                  45
Glu Ala Leu Glu Arg Ala Lys Leu Gln Glu Lys Pro Ser His Val Arg
    50                  55                  60
Ala Ser Gln
65

SEQ ID NO 35:
Asn Ala Lys Asp Glu Cys Ser Glu Phe Arg Asn Tyr Ile Arg Asn Asn
1               5                   10                  15
Glu Leu Ile Cys Pro Arg Glu Asn Asp Pro Val Cys Gly Ala Asp Gly
            20                  25                  30
Lys Phe Tyr Thr Asn Lys Cys Tyr Met Cys Arg Ala Val Phe Leu Thr
        35                  40                  45
Glu Ala Leu Glu Arg Ala Lys Leu Gln Glu Lys Cys Ser His Val Arg
    50                  55                  60
Ala Ser Gln
65

SEQ ID NO 36:
Glu Glu Asp Ser Pro Asp Ser Phe Ser Ser Leu Asp Ser Glu Met Cys
1               5                   10                  15
Lys Asp Tyr Arg Val Leu Pro Arg Ile Gly Tyr Leu Cys Pro Lys Asp
            20                  25                  30
Leu Lys Pro Val Cys Gly Asp Asp Gly Gln Thr Tyr Asn Asn Pro Cys
        35                  40                  45
Met Leu Cys His Glu Asn Leu Ile Arg Gln Thr Asn Thr His Ile Arg
    50                  55                  60
Ser Thr Gly Lys Cys Glu Glu Ser Ser Thr Pro Gly Thr Thr Ala Ala
65                  70                  75                  80
Ser Met Pro Pro Ser Asp Glu
                85

Polypeptides with antagonistic activity against natural CXCR4 polypeptide

SEQ ID NO 37:
Leu Val Arg Tyr Thr Lys Lys Val Pro Gln Val Ser
1               5                   10

SEQ ID NO 38:
Ile Val Arg Tyr Thr Lys Lys Val Pro Gln Val Ser
1               5                   10

SEQ ID NO 39:
Ile Val Arg Trp Thr Lys Lys Val Pro Gln Val Ser
1               5                   10

SEQ ID NO 40:
Ile Val Arg Trp Thr Cys Lys Val Pro Gln Val Ser
1               5                   10

SEQ ID NO 41:
Ile Val Arg Trp Ser Lys Lys Val Pro Cys Val Ser
1               5                   10

SEQ ID NO 42:
Ile Val Arg Trp Ser Lys Lys Val Pro Gln Val Ser
1               5                   10

SEQ ID NO 43:
Ile Met Arg Trp Ser Arg Lys Met Pro Cys Val Ser
1               5                   10

SEQ ID NO 44:
Ile Leu Arg Trp Ser Arg Lys Leu Pro Cys Val Ser
1               5                   10

SEQ ID NO 45:
Ile Leu Arg Trp Ser Arg Lys Met Pro Cys Val Ser
1               5                   10

SEQ ID NO 46:
Ile Leu Arg Trp Thr Arg Lys Met Pro Cys Val Ser
1               5                   10

SEQ ID NO 47:
Ile Leu Arg Trp Ser Arg Lys Met Pro Cys Met Ser
1               5                   10

SEQ ID NO 48:
Ile Leu Arg Trp Ser Arg Lys Phe Pro Cys Val Ser
1               5                   10

SEQ ID NO 49:
Ile Leu Arg Trp Ser Arg Lys Met Pro Cys Phe Ser
1               5                   10

SEQ ID NO 50:
Ile Leu Arg Trp Ser Arg Lys Met Pro Gln Phe Ser
1               5                   10

SEQ ID NO 51:
Ile Val Arg Trp Ser Lys Lys Met Pro Gln Val Ser
1               5                   10

SEQ ID NO 52:
Leu Val Arg Tyr Thr Gln Lys Ala Pro Gln Val Ser
1               5                   10

Natriuretic Peptides

SEQ ID NO 53:
Arg Ser Ser Cys Phe Gly Gly Arg Met Asp Arg Ile Gly Ala Gln Ser
1               5                   10                  15
Gly Leu Gly Cys
            20

SEQ ID NO 54:
Ser Leu Arg Arg Ser Ser Cys Phe Gly Gly Arg Met Asp Arg Ile Gly
1               5                   10                  15
Ala Gln Ser Gly Leu Gly Cys Asn Ser Phe Arg Tyr
            20                  25

SEQ ID NO 55:
Thr Ala Pro Arg Ser Leu Arg Arg Ser Ser Cys Phe Gly Gly Arg Met
1               5                   10                  15
Asp Arg Ile Gly Ala Gln Ser Gly Leu Gly Cys Asn Ser Phe Arg Tyr
            20                  25                  30

## Claims

1. Pharmaceutical composition comprising
a) an amino acid sequence from at least 70% sequence identity to the amino acid sequence of a polypeptide selected from at least one of the 15 domains of the LEKTI polypeptide;
and at least one selected from
b) an amino acid sequence from at least 75% sequence identity to the amino acid sequence of a polypeptide with antagonistic activities against natural CXCR4;
and
c) an amino acid sequence from at least 70% sequence identity to the amino acid sequence of a natriuretic peptide (ANP).

2. The composition of claim 1 wherein the polypeptide LEKTI, the polypeptide with antagonistic activity against natural CXCR4, or the polypeptide ANP independently of each other, have a modification at their respective N-terminal nitrogen atoms which modification forms together with the amino group of the N-terminal amino acid of the peptide a moiety having the structure -NR²R³ wherein R² and/or R³ are independently from each other H or a substituted or unsubstituted acyl alkyl, aryl, aralkyl, cyclo alkyl and heterocyclo alkyl group.

3. The composition of claim 1 or 2 wherein the polypeptide LEKTI, the polypeptide with antagonistic activity against natural CXCR4, or the polypeptide ANP independently of each other, have a modification at their respective C-terminal carboxyl group which modification forms together with the forms together with the carboxyl group of the C-terminal amino acid of the peptide a moiety having the structure -C(O)-O-R¹ or -C(O)-NR²R³ wherein R¹ is a substituted or unsubstituted alkyl, aryl, aralkyl, cyclo alkyl and heterocyclo alkyl group.

4. The composition of any one of claims 1 to 3 wherein the sequence identity to the LEKTI polypeptide amino acid sequence is at least 80%, 90% or 95 %, and/or
the sequence identity to the polypeptide sequence of the polypeptide with antagonistic activity against natural CXCR4 is at least 80% or 90 %, and/or the sequence identity to the natriuretic polypeptide amino sequence is at least 80%, 90% or 95%.

5. The composition of any one of claims 1 to 4 comprising a mutant or derivative of the LEKTI polypeptide, a mutant or derivative of the peptide with antagonistic activity against natural CXCR4 polypeptide, or a mutant or derivative of the natriuretic peptide.

6. The composition of any one of claims 1 to 5 having a biological activity of inhibiting tumour growth by at least 30 vol.-%, preferably by at least 40 vol.-%, more preferably by at least 50 vol.-%, most preferably by at least 90 vol.-%, determined in a patient-derived xenograft lymphoma model, respectively.

7. The composition of any one of claims 1 to 6 wherein the polypeptide LEKTI is selected from the group of polypeptides consisting of Seq ID No 1 - 36.

8. The composition of any one of claims 1 to 7 wherein the polypeptide with antagonistic activity against natural CXCR4 polypeptide is a peptide having the general amino acid sequence written in the single letter code
X¹ X² R X⁴ X⁵ X⁶ K X⁸ P X¹⁰ X¹¹ S,
wherein
X¹ = L or I,
X² = V, M, or L,
X⁴ = Y or W,
X⁵ = T or S,
X⁶ = K, C, R, or Q,
X⁸ = V, M, L, F, or A,
X¹⁰ = Q or C,
X¹¹ = V, M, or F.

9. The composition of any one of claims 1 to 8 wherein the polypeptide with antagonistic activity against natural CXCR4 polypeptide is selected from the group of EPI-X4 polypeptides consisting of Seq ID No 37 - 52.

10. The composition of any one of claims 1 to 9 wherein the natriuretic peptide is selected from the group of polypeptides consisting of Seq ID No 53 - 55.

11. The composition of any one of claims 1 to 10 comprising
a) an amino acid sequence of a polypeptide selected from the group of polypeptides consisting of Seq ID No 1-36;
and at least one selected from
b) an amino acid sequence of a polypeptide selected from the group of EPI-X4 polypeptides consisting of Seq ID No 37-52;
and
c) an amino acid sequence of a polypeptide selected from the group of polypeptides consisting of Seq ID No 53 - 55.

12. The composition of any one of claims 1 to 11 formulated for intravascular, lymphatic, intracardial, parenteral, intravenous, intramuscular, intrathecal, pulmonary, epi-, intra- and sub-cutaneous, intranasal, ophthalmic, auricular, buccal, or intraperitoneal administration, or for local administration to an organ, or for administration into the cerebrospinal fluid space.

13. The composition of any one of claims 1 to 12 comprising pharmaceutically acceptable carriers, stabilizers, and/or auxiliaries.

14. The composition of any one of the claims 1 to 13 for use in the treatment of neurological diseases, in particular stroke, Parkinson's disease, Alzheimer's disease, or multiple sclerosis; in the treatment of immunologic disorders, in particular in the treatment of the WHIm-syndrom, lupus erythematosus or rheumatoid arthritis; in the treatment of cancers, in particular sarcomas, lymphoma, leukaemia, or cancers showing the CRCX receptor such as cancer of the liver, pancreas, prostate, or breast cancer; in the treatment of dermatological diseases or inflammatory skin syndromes, in particular atopic dermatitis; in the treatment of lack of mobilization, proliferation and migration of stem cells, T-cell activation as well as support of immunoblasts such as CTL/PD-1; in the treatment of wounds caused by burning; in the treatment of antifibrosis; in the treatment or prevention of scars; in pain management; in the treatment of cardiologic disorders, in particular heart insufficiency; in the treatment of metabolic disorders, in particular diabetes; in the treatment of viral diseases, in particular COVID-19, infections with SARS-CoV-2, HIV-I, HIV-2, *Cytomegalo virus, Herpes simplex virus (type 1 and 2), Varicella zoster virus, Hepatitis A and Hepatitis B virus, Influenza virus, Polio virus, Rhino virus, Rubella virus, Measles virus, Rabies virus, Rous sarcoma virus,* or *Epstein-Barr Virus;* in the treatment of infections caused by bacteria and fungi, in particular *Pseudomonas, Candida,* or S. *aureus;* in the treatment of infectious processes or abnormal infectious processes; in the treatment of cervical inflammations, inflammations of the Bartholinian glands and other vaginal areas, tonsillitis, pharyngitis and laryngitis, acute or chronic inflammatory processes associated with excessive mucus formation and resulting acute emergency situations, or postoperative bleeding due to hyperfibrinolysis; in the prophylaxis of pulmonary emphysema formation in a1-proteinase inhibitor deficiency; in the treatment of asthma or pneumonia; in the treatment of growth disorders; in the treatment of neuronal diseases, disorders of the blood clotting cascade and hematopoiesis, vascular diseases, diseases of the immune system, organ infarcts, peripheral circulatory disorders, renal insufficiency, muscular degeneration, degenerative-genetic or acquired diseases, bone diseases, or in improving wound and bone healing.
